# EUROPEAN PATENT APPLICATION

(11) **EP 3 351 092 A1**
(43) Date of publication of application: **25.07.2018**
(21) Application number: 16865941.5
(22) Date of filing: 05.01.2016
(51) Int. Cl.: A01K 1/015

(54) **EXCREMENT DISPOSAL SHEET FOR ANIMALS**

(30) Priority: 19.11.2015 JP 2015226921; 25.12.2015 JP 2015254249
(71) Applicant: Unicharm Corporation, Ehime 799-0111 (JP)
(72) Inventor: TAKAHASHI, Yumei, Kanonji-shi, Kagawa 769-1602 (JP); SASANO, Yasuhiro, Kanonji-shi, Kagawa 769-1602 (JP)
(74) Representative: Dolleymores
(86) International application number: PCT/JP2016/050142
(87) International publication number: WO 2017/085937

(57) **Abstract**

The present invention addresses the problem of providing an excrement disposal sheet for animals, of which a surface sheet can exhibit sufficiently superior and enough concealing performance without any structural constraint. The excrement disposal sheet for animals (1) according to the present invention is provided with a liquid-permeable front surface seat (2) composed of white fibers, a liquid-impermeable back surface sheet (4) and an absorbent material (3) arranged between these sheets, wherein the front surface seat (2) has a first surface that is a surface onto which excrement is to be supplied and a second surface that is opposed to the first surface, and the first surface in the front surface sheet (2) has an average absorbance of 700 or more, a floc average value of 6.0 or less and a floc ratio of 0.6 to 1.7.

## Description

### Technical Field

The present invention relates to an excreta treatment sheet for animals to be used when rearing animals such as dogs.

### Background Art

As excreta treatment sheets for animals, which are used for treatment of excreta that have been excreted by pets such as dogs, there are known excreta treatment sheets for animals comprising a liquid-permeable top sheet, a liquid-impermeable back sheet, and an absorbent body disposed between the sheets.

Among these there are also known excreta treatment sheets for animals having a satisfactory masking property for excreta that have been excreted from pets. As an example of an excreta treatment sheet for animals having such a satisfactory masking property, PTL 1 proposes an absorbent sheet for pets comprising a liquid-permeable top sheet, a liquid-impermeable back sheet, an absorbent body situated between the sheets, and an interlayer sheet disposed between the top sheet and the absorbent body, the absorbent sheet for pets having colored regions that are visible from the excretion side and that form a color pattern together with other regions of the absorbent sheet for pets, the top sheet being formed with a lower total light transmittance than the interlayer sheet.

The absorbent sheet for pets disclosed in PTL 1 having the colored regions as described above can conceal excreta held in the interlayer sheet and the absorbent body, while the lower total light transmittance of the top sheet compared to the interlayer sheet makes excreta held in the interlayer sheet and absorbent body less visible through the top sheet.

### Citation List

### Patent Literature

[PTL 1] Japanese Unexamined Patent Publication No. 2014-117177

### Summary of Invention

### Technical Problem

However, in order to obtain a satisfactory masking property with the absorbent sheet for pets disclosed in PTL 1, it is designed with structural restrictions including the need to at least have the colored regions while satisfying the relationship between the specific total light transmittances of the top sheet and the interlayer sheet, and depending on the color pattern of the colored regions, and in cases where they do not match the color of the excreta held in the interlayer sheet or absorbent body, it has not been possible to adequately conceal the excreta.

In addition, when the total light transmittance of the top sheet is merely lower than that of the interlayer sheet, as with the absorbent sheet for pets disclosed in PTL 1, even if the color of the excreta held in the interlayer sheet or absorbent body can be relatively faintly seen, any irregularities in the structure, such as irregularities in fiber density, in the in-plane direction of the top sheet (i.e., irregularities in light permeability) will cause the boundaries between the portions where the excreta are present and the portions where the excreta are not present to be visible in the interlayer sheet and the absorbent body, and this has sometimes made it impossible to adequately conceal excreta held in the interlayer sheet or absorbent body.

It is therefore an object of the present invention to provide an excreta treatment sheet for animals that can exhibit an adequately excellent masking property with just a top sheet alone, without the structural restrictions mentioned above.

### Solution to Problem

One aspect (aspect 1) of the invention is an excreta treatment sheet for animals comprising a liquid-permeable top sheet composed of white fibers, a liquid-impermeable back sheet and an absorbent body disposed between the sheets, wherein the top sheet has a first surface serving as an excreta supply surface and a second surface that is the surface on the side opposite the first surface, and the top sheet has a mean absorbance of 700 or greater on the first surface, a mean flock value of no greater than 6.0 on the first surface, and a flock ratio of 0.6 to 1.7 on the first surface.

Since the excreta treatment sheet for animals of aspect 1 has a mean absorbance of 700 or greater and a strong degree of whiteness (i.e. concentration of whiteness) on the first surface of the top sheet, the color of excreta such as urine that has been absorbed and held in the absorbent body through the top sheet is less likely to be visible on the top sheet side. In addition, since the excreta treatment sheet for animals of aspect 1 has a mean flock value of no greater than 6.0 on the first surface of the top sheet and a flock ratio of 0.6 to 1.7 on the first surface, there is less color irregularity of whiteness on the first surface of the top sheet (i.e., the first surface is uniformly white), and the boundaries between the portions where excreta are present and the portions where excreta are not present in the absorbent body are less visible from the top sheet side.

Consequently, in the excreta treatment sheet for animals of aspect 1, not only is the color of the excreta itself held in the absorbent body less visible, but the outlines of the portions holding the excreta in the absorbent body are also less visible, and therefore a sufficiently excellent masking property can be exhibited simply by providing a top sheet satisfying the aforementioned specific requirements.

According to another aspect (aspect 2) of the invention, in the excreta treatment sheet for animals of aspect 1, the mean absorbance is no greater than 2000 on the first surface of the top sheet.

Since the excreta treatment sheet for animals of aspect 2 has a mean absorbance of no greater than 2000 on the first surface of the top sheet, and excreta held in the absorbent body are therefore not completely concealed, the owner of an animal (for example, a dog) can confirm the presence or absence of animal excreta, even though it is poorly visible, and can realize that it is time to exchange the excreta treatment sheet.

According to yet another aspect (aspect 3) of the invention, in the excreta treatment sheet for animals according to aspect 1 or 2, the top sheet has an uneven structure on the first surface and a flat structure on the second surface.

Since the excreta treatment sheet for animals of aspect 3 has an uneven structure on the first surface of the top sheet serving as the excreta supply surface, and the members (such as the absorbent body) located on the absorbent body side of the top sheet at the raised sections and recesses of the uneven structure have different visibilities, the outlines of the portions of the absorbent body where excreta are present are even less likely to be visible. Moreover, since the second surface of the top sheet facing the absorbent body has a flat structure and therefore gaps are less likely to form between the second surface of the top sheet and the absorbent body, animal excreta supplied onto the first surface of the top sheet are readily incorporated into the absorbent body side by capillary phenomenon, allowing liquid residue of excreta on the top sheet to be prevented.

Therefore, traces of excreta on the top sheet of the excreta treatment sheet for animals of aspect 3 are less likely to be visible.

According to yet another aspect (aspect 4) of the invention, in the excreta treatment sheet for animals according to any one of aspects 1 to 3, the white fibers include titanium oxide, the content of the titanium oxide in the fibers being 1 to 3 mass%.

In the excreta treatment sheet for animals of aspect 4, the white fibers include a prescribed amount of titanium oxide which is a white pigment with a particularly high refractive index, allowing a top sheet satisfying the aforementioned ranges for the absorbance and flock ratio of the first surface to be more easily and reliably obtained, and therefore the aforementioned excellent masking property can be more stably and reliably exhibited.

According to yet another aspect (aspect 5) of the invention, in the excreta treatment sheet for animals of aspect 4, the white fibers are composed of core-sheath thermoplastic resin fibers, and the titanium oxide is present only in the cores of the core-sheath thermoplastic resin fibers.

In the excreta treatment sheet for animals of aspect 5, the titanium oxide is present only in the cores of the core-sheath thermoplastic resin fibers composing the white fibers, and therefore when incident light on the fibers is reflected by the titanium oxide in the cores, the reflected light is refracted at the sheaths covering the outer surfaces of the cores and exits the fibers, resulting in more diffuse reflection of the light.

Consequently, the excreta treatment sheet for animals of aspect 5 can exhibit a more excellent masking property for the top sheet.

In addition, when the top sheet is composed of such specific core-sheath thermoplastic resin fibers (i.e., core-sheath thermoplastic resin fibers wherein titanium oxide is present only in the cores), joining between the fibers by heat sealing of the sheaths is less likely to be inhibited by the titanium oxide during production of the top sheet, and therefore the fibers can be more reliably joined together and the top sheet can be given more excellent strength. Thus, the cores of the core-sheath thermoplastic resin fiber constituents of the top sheet are less likely to be affected by scratching behavior of the animal, so that the function and effect of the titanium oxide can be exhibited more persistently for a longer period of time.

According to another aspect (aspect 6) of the invention, in the excreta treatment sheet for animals of any one of aspects 1 to 5, the thickness of the top sheet is 0.5 mm or greater.

Since in the excreta treatment sheet for animals of aspect 6, the thickness of the top sheet is 0.5 mm or greater and the first surface of the top sheet (i.e., the excreta supply surface) and the absorbent body holding the excreta are separated by a fixed degree, excreta held in the absorbent body is even less likely to be visible.

According to another aspect (aspect 7) of the invention, in the excreta treatment sheet for animals of any one of aspects 1 to 6, the basis weight of the top sheet is 20 g/m ² or greater.

Since in the excreta treatment sheet for animals of aspect 7 the basis weight of the top sheet is 20 g/m² or greater and the interfiber distance between the fibers composing the top sheet is within a prescribed range, the absorbent body holding excreta is even less likely to be visible due to compacting of the fibers, and capillary phenomenon causing the excreta to migrate from the first surface to the second surface can be even more effectively exhibited in the top sheet. This allows the excreta treatment sheet for animals of aspect 7 to exhibit even more excellent masking performance and high absorption (absorption rate).

According to yet another aspect of the invention (aspect 8), in the excreta treatment sheet for animals according to any one of aspects 1 to 7, the absorbent body has an absorbent core including an absorbent material, and a colored sheet at least partially having colored regions is disposed between the top sheet and the absorbent core.

Since the excreta treatment sheet for animals of aspect 8 comprises the colored sheet between the top sheet and the absorbent core, in addition to the aforementioned concealing effect of the top sheet, excreta held in the absorbent core of the absorbent body blends in with the color shade and pattern in the colored regions of the colored sheet and is less conspicuous, such that excreta held in the absorbent body (especially the absorbent core) is even less likely to be visible.

### Advantageous Effects of Invention

According to the invention it is possible to provide an excreta treatment sheet for animals that, without having such structural restrictions, can exhibit an adequately excellent masking property with just a top sheet alone.

### Brief Description of Drawings

Fig. 1 is a perspective view of an excreta treatment sheet for animals according to one embodiment of the invention.
Fig. 2 is a cross-sectional view of an excreta treatment sheet for animals according to one embodiment of the invention, along line II-II of Fig. 1.
Fig. 3(a) is a plan view schematically showing the state of an excreta treatment sheet for animals according to one embodiment of the invention after absorption of urine, and Fig. 3(b) is a plan view schematically showing the state of an excreta treatment sheet for animals of the prior art after absorption of urine.
Fig. 4(a) is a plan view of an excreta treatment sheet for animals according to another embodiment of the invention as viewed from the top sheet side, and Fig. 4(b) is a plan view schematically showing the state of the excreta treatment sheet for animals of Fig. 4(a) after absorption of urine.

### Description of Embodiments

Preferred embodiments of the excreta treatment sheet for animals according to the invention will now be described in detail with reference to the accompanying drawings. Throughout the present specification, unless otherwise specified, the concept of "an object (for example, an excreta treatment sheet for animals, a top sheet or an absorbent body) situated on the horizontal plane in the spread open state with the excreta supply surface facing upward, being viewed in the thickness direction of the object from the top side in the vertical direction" will be referred to simply by the phrase "in the planar view", and a drawing in the planar view will be referred to as a "plan view".

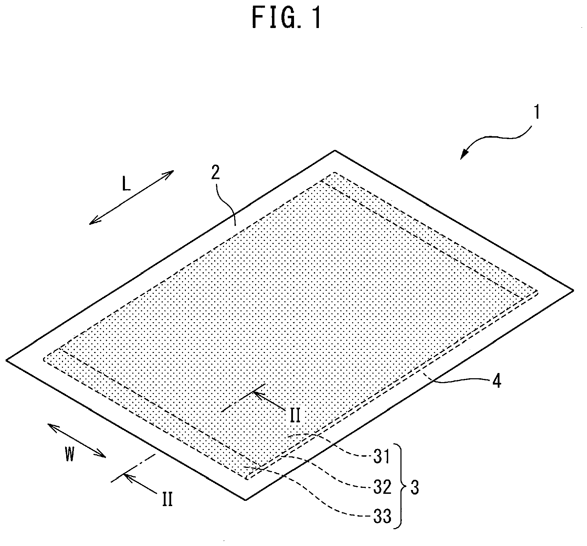

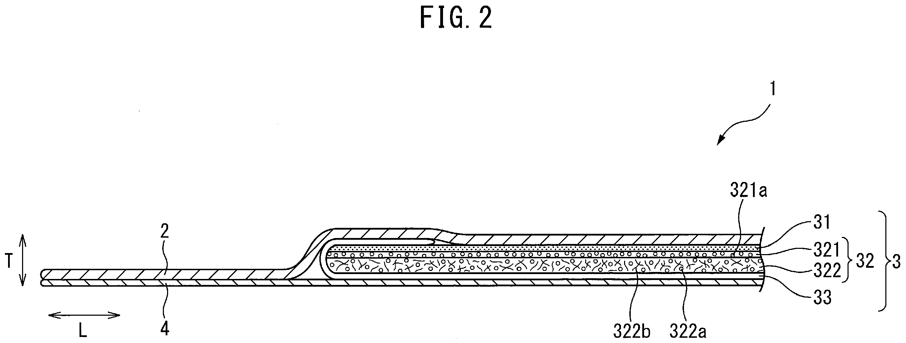

Fig. 1 is a perspective view of an excreta treatment sheet for animals 1 according to one embodiment of the invention, and Fig. 2 is a cross-sectional view of the excreta treatment sheet for animals 1 along line II-II in Fig. 1.

As shown in Fig. 1, the excreta treatment sheet for animals 1 according to one embodiment of the invention is a rectangular sheet, in the planar view, having a lengthwise direction L, a widthwise direction W and a thickness direction T. According to the invention, the outer shape of the excreta treatment sheet for animals is not limited to such a rectangular shape, and any desired shape such as square, polygonal, circular or elliptical may be employed, depending on the purpose of use, design, etc. Furthermore, the size of the excreta treatment sheet for animals 1 may be set as appropriate depending on the size and type of animal to which the excreta treatment sheet is to be applied, and when the animal is a dog, for example, the length in the lengthwise direction L is about 400 mm to 1200 mm and the length in the widthwise direction W is about 250 mm to 800 mm.

As shown in Fig. 1, the excreta treatment sheet for animals 1 is set at a prescribed location of a rearing space for an animal such as a pet (for example, indoors) in the spread out state, and used to absorb and hold excreta such as urine that have been excreted from an animal, in order to keep the rearing space in a clean condition. Incidentally, the excreta treatment sheet for animals 1 may be set directly on the floor surface or ground of the rearing space, or it may be set via a prescribed holder, tray, mat or the like.

The "animal" to which the excreta treatment sheet for animals according to the invention is to be applied is not particularly restricted so long as it is an animal that can be reared, such as a pet, and it may be a mammal such as a dog, cat or hamster, or any of various other types of animals such as a bird, reptile or amphibian.

According to the invention, the "excreta" to be absorbed and held by the excreta treatment sheet for animals are not particularly restricted so long as they can be absorbed and held in the absorbent body described below, and for example, they may be various body fluids, including liquid to low-viscosity body feces, or urine, mouth excretions such as saliva, or blood.

As shown in Fig. 1 and Fig. 2, the excreta treatment sheet for animals 1 according to this embodiment comprises, in the thickness direction T, a liquid-permeable top sheet 2 forming the supply surface for excreta that have been excreted from an animal, a liquid-impermeable back sheet 4 forming the mounting surface of the excreta treatment sheet for animals 1, which is the surface on the opposite side from the excreta supply surface, (i.e., the surface facing the floor surface or ground on which the excreta treatment sheet for animals has been set), and a liquid-absorbing absorbent body 3 disposed between these sheets, the top sheet 2 and the back sheet 4 being mutually joined by any desired joining means such as a hot-melt adhesive around their respective perimeters.

For this embodiment, the top sheet 2 is composed of white fibers, and has a first surface serving as the supply surface for excreta that have been excreted from an animal, and a second surface that is the surface on the side opposite the first surface, facing the absorbent body 3, the mean absorbance being 700 or greater on the first surface, the mean flock value being no greater than 6.0 on the first surface, and the flock ratio being 0.6 to 1.7 on the first surface (hereunder, the requirements related to the mean absorbance, mean flock value and flock ratio may also be referred to simply as "specific requirements").

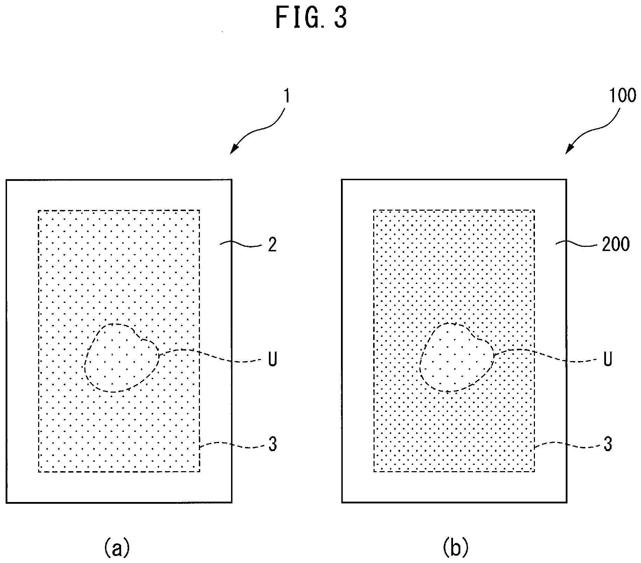

Fig. 3(a) is a plan view schematically showing the state of an excreta treatment sheet for animals 1 according to this embodiment after absorption of urine as excreta, and Fig. 3(b) is a plan view schematically showing the state of an excreta treatment sheet for animals 100 of the prior art (i.e., using as the top sheet a top sheet 200 that does not satisfy the specific requirements mentioned above), after absorption of urine.

In the excreta treatment sheet for animals 1 of this embodiment, since the mean absorbance is 700 or greater on the first surface of the top sheet 2 and the degree of whiteness is strong (i.e., deep whiteness), the color of excreta such as urine U (excreta) that has been absorbed and held in the absorbent body 3 through the top sheet 2 is less likely to be visible on the top sheet side, as shown in Fig. 3(a). In addition, since it has a mean flock value of no greater than 6.0 on the first surface of the top sheet 2 and a flock ratio of 0.6 to 1.7 on the first surface (i.e., low textural irregularity of the top sheet 2), the whiteness on the first surface of the top sheet 2 is less irregular (or in other words, the first surface is uniformly white), and the boundaries between the portions where urine U is present and the portions where urine U is not present in the absorbent body 3 are less likely to be visible from the top sheet 2 side, as shown in Fig. 3(a).

Consequently, in the excreta treatment sheet for animals 1 of this embodiment, not only is the color of the urine U itself being held in the absorbent body 3 less visible, as shown in Fig. 3(a), but the outlines of the portions holding the urine U in the absorbent body 3 are also less visible, and therefore a sufficiently excellent masking property can be exhibited simply by providing a top sheet 2 satisfying the aforementioned specific requirements.

However, since these specific requirements are not satisfied by the top sheet 200 in the excreta treatment sheet for animals 100 of the prior art, the color of the urine U itself held in the absorbent body 3 is more visible, as shown in Fig. 3(b), and the outlines of the portions holding the urine U in the absorbent body 3 are also more visible.

Here, the term "mean absorbance" is a physical value representing the degree to which the intensity of light passing through an object (for example, a top sheet) is attenuated by absorption, scattering, reflection and the like, and it is expressed as the logarithm of the ratio of the intensity of the incident beam when light strikes the object and the intensity of the transmitted light when the light exits the object.

Therefore, according to the invention, a larger mean absorbance on the first surface of the top sheet composed of white fibers means that light is scattered and reflected more easily and the degree of whiteness becomes stronger (i.e., the whiteness becomes deeper), and therefore the color of excreta such as urine absorbed and held by the absorbent body is less likely to be visible from the first surface side of the top sheet.

The mean absorbance can be measured using a Formation Tester (FMT-MIII) by Nomura Shoji Co., Ltd., for example, under the measuring conditions described below.

Throughout the present specification, "flock" is a value for the sample surface photographed with a CCD camera and digitized using the mean transmittance as the threshold value, with pixels below the threshold value as "black" and pixels above the threshold value as "white", to allow evaluation of the uniformity (or textural irregularity) of the fiber distribution of the top sheet.

Throughout the present specification, the digitized value, obtained by scanning all of the pixels of the photographed sample surface in the horizontal direction to determine the total number of black pixels (lower transmittance than the threshold value), and dividing this by the number of groups of one or more black pixels in continuous contact, is referred to as the "flock value", the flock value in the lengthwise direction L being more specifically referred to as the "first direction flock value" and the flock value in the widthwise direction as the "second direction flock value". However, when the top sheet does not have a long shape with a lengthwise direction and a widthwise direction as with this embodiment, the flock value in either arbitrary direction will be referred to as the "first direction flock value" and the flock value in the other direction perpendicular to the arbitrary direction on the same plane will be referred to as the "second direction flock value".

Also throughout the present specification, the average value of the first direction flock value and the second direction flock value will be referred to as the "mean flock value", and the value of the smaller of the first direction flock value and the second direction flock value divided by the larger of the flock values will be referred to as the "flock ratio". The mean flock value is an index representing the degree of uniformity of absorbance for the entire top sheet, and the flock ratio is an index representing the bias of absorbance in the two prescribed directions (i.e., the lengthwise direction L and widthwise direction W).

According to the invention, therefore, a top sheet composed of white fibers that has a smaller mean flock value and a flock ratio closer to 1 has a more even distribution without maldistribution of the white fibers composing the top sheet, and less irregularity of white color on the first surface of the top sheet, such that the boundaries between the portions of the absorbent body where excreta such as urine are present and the portions where excreta are not present are less visible from the first surface side of the top sheet.

The mean flock value and the flock ratio can be determined by measuring the flock values in the lengthwise direction L and the widthwise direction W (i.e., the first direction flock value and the second direction flock value) under the measuring conditions described below, using a Formation Tester (FMT-MIII) by Nomura Shoji Co., Ltd. as mentioned above, and calculating from the flock values.

Each of the members composing the excreta treatment sheet for animals 1 of this embodiment will now be described in greater detail.

As shown in Fig. 1 and Fig. 2, the top sheet 2 is composed of a sheet-like member that is disposed at a location in the thickness direction T of the excreta treatment sheet for animals 1 on the side where the excreta from an animal is supplied, and that has liquid permeability allowing excreta that has been excreted from the animal to be moved from the excreta supply surface (i.e., the first surface) to the opposite side (i.e., the second surface). The liquid-permeable sheet-like member composing the top sheet 2 is not particularly restricted so long as the sheet-like member is composed of white fibers and satisfies the specific requirements mentioned above, and any nonwoven fabric such as an air-through nonwoven fabric, spunbond nonwoven fabric or point bond nonwoven fabric may be suitably used. The nonwoven fabric may also be subjected to hydrophilicizing treatment with a surfactant.

Among these, the liquid-permeable sheet-like member is preferably an air-through nonwoven fabric from the viewpoint of easier formation of a top sheet satisfying the specific requirements mentioned above.

The white fibers composing the top sheet 2 are not particularly restricted so long as the fibers exhibit a white color, and suitable examples to be used include thermoplastic resin fibers to which white pigment or white dye has been added, and thermoplastic resin fibers having a surface structure or crystalline structure capable of causing diffuse reflection of light.

Throughout the present specification, "white color" means an L* value of 80 or greater according to the L*a*b color system.

Among such white fibers, the white fibers composing the top sheet are preferably thermoplastic resin fibers with a white pigment added, from the viewpoint of easily forming a top sheet satisfying the aforementioned specific requirements.

The white pigment to be added to the thermoplastic resin fibers is not particularly restricted and may be, for example, titanium oxide (titanium white), zinc oxide, a mixture of barium sulfate and zinc sulfide or basic lead carbonate (lead white), the white pigment preferably being titanium oxide from the viewpoint of a high refractive index and low toxicity for the living body.

When titanium oxide is used as the white pigment, the titanium oxide content in the thermoplastic resin fibers is not particularly restricted but is preferably 1 to 3 mass%. If the white fibers composing the top sheet include 1 to 3 mass% of titanium oxide which has a particularly high refractive index among white pigments, then a top sheet satisfying the aforementioned specific requirements can be more easily and reliably obtained, and therefore the aforementioned excellent masking property of the top sheet can be more stably and reliably exhibited.

The type of thermoplastic resin fibers are not particularly restricted so long as they are fibers made of a thermoplastic resin, and examples for the thermoplastic resin include publicly known resins including olefin-based resins such as polyethylene (PE), polypropylene (PP) and ethylene-vinyl acetate copolymer (EVA), polyester-based resins such as polyethylene terephthalate (PET) and polylactic acid (PLA), and polyamide-based resins such as 6-nylon, any of which resins may be used alone or as combinations of two or more of such resins.

The structure of the fibers made of such thermoplastic resins is not particularly restricted, and examples include composite fibers such as core-sheath fibers, side-by-side fibers and island/sea fibers; hollow type fibers; modified cross-section fibers such as flat, Y-shaped or C-shaped fibers; solid crimped fibers with latent crimping or developed crimping; and splittable fibers that are split by a physical load such as a water stream, heat or embossing, and fibers having such structures may be used alone, or two or more different types may be used in combination.

Among such thermoplastic resin fibers, it is preferred to use core-sheath thermoplastic resin fibers such as polyester (core)/polyethylene (sheath) fibers, and it is particularly preferred to use core-sheath thermoplastic resin fibers having the titanium oxide present only in the core.

If the titanium oxide is present only in the cores of the core-sheath thermoplastic resin fibers, then when incident light on the fibers is reflected by the titanium oxide in the cores, the reflected light will be refracted at the sheaths covering the outer surfaces of the cores and will exit the fibers, resulting in more diffuse reflection of the light, and therefore fibers with a strong degree of whiteness can be obtained even with a low titanium oxide content.

In addition, when the top sheet is composed of such specific core-sheath thermoplastic resin fibers (i.e., core-sheath thermoplastic resin fibers wherein titanium oxide is present only in the cores), joining between the fibers by heat sealing of the sheaths is less likely to be inhibited by the titanium oxide during production of the top sheet, and therefore the fibers can be more reliably joined together and the top sheet can be given more excellent strength. As a result, the cores of the core-sheath thermoplastic resin fiber constituents of the top sheet are less likely to be affected by scratching behavior of the animal, such that the function and effect of the titanium oxide can be exhibited more persistently for a longer period of time.

The size of the thermoplastic resin fibers is not particularly restricted, and for example, a size range of 1.1 to 8.8 dtex may be mentioned, but from the viewpoint of nonwoven fabric strength, flexibility and liquid permeability, it is preferably in the range of 1.5 to 4.6 dtex.

The thermoplastic resin fibers may also be subjected to hydrophilicizing treatment by, for example, treatment utilizing a surfactant, hydrophilic agent or the like (for example, kneading of a surfactant into the fiber interiors, or coating of the fiber surfaces with a surfactant).

In the excreta treatment sheet for animals 1 of this embodiment, the top sheet 2 is preferably composed of white fibers alone from the viewpoint of masking property; however, other non-white fibers or optional additives may also be included in ranges that do not inhibit the effect of the invention.

Such other fibers may be fibers comprising the same thermoplastic resin as the thermoplastic resin mentioned above, their contents preferably being no greater than 75 mass% of the total fibers composing the top sheet.

Examples of optional additives include aromatics, and deodorants, antimicrobial agents, microbicides and the like. Such additives may also be used in combinations of two or more different types.

In the excreta treatment sheet for animals 1 of this embodiment, the top sheet 2 has a mean absorbance of 700 or greater and preferably 800 or greater on the first surface serving as the excreta supply surface, as mentioned above.

There is no particular upper limit for the mean absorbance on the first surface, from the viewpoint of the masking property, but it is preferably no greater than 2000 and more preferably no greater than 1800.

If the mean absorbance is no greater than 2000 on the first surface of the top sheet 2, excreta held in the absorbent body 3 will not be completely concealed, and therefore the owner of an animal (for example, a dog) will be able to confirm the presence or absence of animal excreta, even though it is poorly visible, and will be able to accurately determine that it is time to exchange the excreta treatment sheet.

The means for controlling the mean absorbance of the top sheet (for example, to a mean absorbance of 700 or greater) is not particularly restricted, and for example, it may be adjustment of the white pigment content in the fibers composing the top sheet, or using thermoplastic resin fibers with a surface structure or crystalline structure that readily causes diffuse reflection of light, as the fibers composing the top sheet.

As mentioned above, the top sheet 2 has a mean flock value of no greater than 6.0 on the first surface serving as the excreta supply surface, and a flock ratio of 0.6 to 1.7 on the first surface. The mean flock value and flock ratio are more preferably no greater than 5.0, and 0.7 to 1.4, respectively.

The means for controlling the mean flock value and flock ratio of the top sheet (for example, limiting the mean flock value to no greater than 6.0 and the flock ratio to 0.6 to 1.7) is not particularly restricted, and for example, it may be appropriate selection or adjustment of the different constituent features, including the type of nonwoven fabric composing the top sheet (for example, an air-through nonwoven fabric or the like), or its basis weight or thickness, the type of thermoplastic resin fibers (for example, core-sheath thermoplastic resin fibers or the like), or the white pigment content.

In addition, the top sheet 2 preferably has an uneven structure in which raised sections and recesses are irregularly situated over essentially the entire surface of the first surface, and most preferably it also has a flat structure on the second surface. If the first surface of the top sheet has such an irregular uneven structure, the visibility of the absorbent body located on the second surface side of the top sheet will differ at the raised sections and recesses of the uneven structure, and therefore the outlines of the portions of the absorbent body where excreta are present are even less likely to be visible. Moreover, if the first surface (i.e., the excreta supply surface) of the top sheet 2 has such an uneven structure, the uneven structure will increase the friction resistance between the foot soles of an animal such as a dog stepping onto the excreta treatment sheet for animals 1, and the first surface of the top sheet 2, thus helping to prevent slipping on the top sheet 2 when the animal excretes, and allowing excretion to be carried out without a sense of stress.

Moreover, when the second surface of the top sheet facing the absorbent body has a flat structure, gaps are less likely to form between the second surface of the top sheet and the absorbent body (i.e., the second surface of the top sheet and the absorbent body tightly contact together more easily), and therefore animal excreta supplied onto the first surface of the top sheet will be readily incorporated into the absorbent body side by capillary phenomenon and the absorption rate of the excreta can be increased, allowing liquid residue of excreta on the top sheet to be prevented.

Therefore, an excreta treatment sheet for animals that uses such a top sheet is less likely to have visible traces of excreta on the top sheet.

According to this embodiment, the thickness of the top sheet 2 is not particularly restricted so long as it satisfies the aforementioned specific requirements, but it is preferably 0.5 mm or greater. If the thickness of the top sheet is 0.5 mm or greater, the first surface (i.e., the excreta supply surface) of the top sheet and the absorbent body holding the excreta will be separated by a fixed degree (for example, 0.5 mm or greater), and therefore excreta held in the absorbent body will be even less likely to be visible.

The thickness of the top sheet can be measured in the following manner using a COMPRESSION TESTER (model: KES-FB3 AUTO-A by Kato Tech Corp.) as the measuring apparatus, for example. For measurement of the thickness of the top sheet, a measuring sample taken from the top sheet is placed in the measuring apparatus and the thickness is measured with a pressure of 0.5 g/cm² applied to the measuring sample with measuring terminals. The thickness of the top sheet is defined as the average value of N = 10 measured values.

The basis weight of the top sheet 2 for this embodiment is not particularly restricted so long as the aforementioned specific requirements are satisfied, but it is preferably 20 g/m ² or greater, and most preferably 22 g/m² to 25 g/m². If the basis weight of the top sheet is 20 g/m² or greater, then the interfiber distance between the fibers composing the top sheet will be within the prescribed range, and therefore the absorbent body holding excreta will be even less likely to be visible, due to compacting of the fibers, and capillary phenomenon causing the excreta to migrate from the first surface to the second surface will be even more effectively exhibited in the top sheet.

Consequently, an excreta treatment sheet for animals using such a top sheet will be able to exhibit even more excellent masking performance and high absorption (absorption rate).

The absorbent body 3 of the excreta treatment sheet for animals 1 of this embodiment will now be described in greater detail.

As shown in Fig. 2, the absorbent body 3 is an absorbing member that is disposed on the side (i.e., on the second surface side) of the top sheet 2 that is opposite the excreta supply surface (i.e., the first surface), and that absorbs and holds excreta that have permeated the top sheet 2. The top sheet 2 and the absorbent body 3 are joined by an optional adhesive such as a hot-melt adhesive, the adhesive being disposed between the top sheet 2 and the absorbent body 3 to a basis weight level that does not interfere with permeation of excreta (for example, 0.1 to 10 g/m²), and provided in a coated state (for example, a spiral, dotted or striped form).

For this embodiment, incidentally, as shown in Fig. 1 and Fig. 3, the absorbent body 3 has an essentially rectangular outer shape in the planar view and is disposed in the center region of the excreta treatment sheet for animals 1; however, according to the invention there is no limitation to this aspect, and the absorbent body may have any desired shape other than rectangular, in the planar view, and it may also be disposed at a location shifted from the center of the excreta treatment sheet for animals in any direction.

According to this embodiment, as shown in Fig. 2, the absorbent body 3 is composed of a hydrophilic colored sheet 31 located on the top sheet side and colored with an optionally selected color such as blue, green, yellow, orange or red, an absorbent core 32 located on the back sheet side and formed by an absorbent material for absorption and holding of excreta, and a core wrap sheet 33 comprising at least one liquid-permeable sheet, covering the colored sheet 31 and the absorbent core 32 from the back sheet side.

In addition, as shown in Fig. 2, the absorbent core 32 is composed of a first absorbent core 321 located on the top sheet side and formed of an absorbent polymer 321a as an absorbent material, and a second absorbent core 322 located on the back sheet side and formed of an absorbent polymer 322a and water-absorbent fibers 322b as absorbent materials.

The colored sheet 31 is composed of tissue paper colored with an optionally selected color such as blue, green, yellow, orange or red (for example, tissue paper with a basis weight of 13.5 g/m², formed of Northern bleached Kraft pulp as the main material), and it is disposed in the absorbent body 3 so as to cover the surface of the absorbent core 32 on the top sheet side, to prevent deformation of the absorbent core 32. When the colored sheet 31 is colored with such a color, the color of excreta held in the absorbent core 32 will blend with the color shade of the colored sheet 31 to become less conspicuous, and therefore excreta held in the absorbent body 3 will be even less visible from the top sheet side.

For this embodiment, incidentally, the colored sheet 31 and the absorbent core 32 are bonded by water spraying. The water spraying is bonding means in which water is sprayed onto the top surface of the absorbent core 32 (specifically, the first absorbent core 321 composed of the absorbent polymer 321a) (i.e., the surface of the excreta treatment sheet for animals on the top sheet side) during production of the absorbent body 3, and the colored sheet 31 is attached onto it. The sprayed water is then evaporated off to leave the water-sprayed sections in a dry state, so that the colored sheet 31 and the absorbent core 32 (specifically, the first absorbent core 321 composed of the absorbent polymer 321a) are in a directly joined state. When the colored sheet 31 and absorbent core 32 are bonded by water spraying in this manner, the excreta such as urine that has migrated from the top sheet side can be rapidly absorbed into the absorbent core 32 without adhesive lying between the colored sheet 31 and the absorbent core 32, so that the aforementioned concealing effect or deodorant effect can be exhibited immediately after excretion by an animal, and a more comfortable rearing environment can be provided for the owner of the animal.

For this embodiment, the entire colored sheet 31 is colored with an optionally selected color such as blue, green, yellow, orange or red; however, the present invention is not restricted to this aspect, and it is sufficient if the colored sheet has at least partially colored regions.

Such colored regions may be composed of a single color shade or different types of color shades, and the colored regions may be created in any desired form such as a pattern, figure, or characters, using the color shades.

When the colored regions of the colored sheet are created in the form of a pattern, figures or characters using the color shades, the outlines of excreta held in the absorbent core (i.e., the boundaries between the portions where excreta are present and the portions where excreta are not present in the absorbent core) blend in with the pattern or figures etc. of the colored sheet and become less conspicuous, such that excreta held in the absorbent body become even less visible from the top sheet side.

The coloration means for formation of the colored regions is not particularly restricted, and any desired coloration means such as dyeing or printing may be employed.

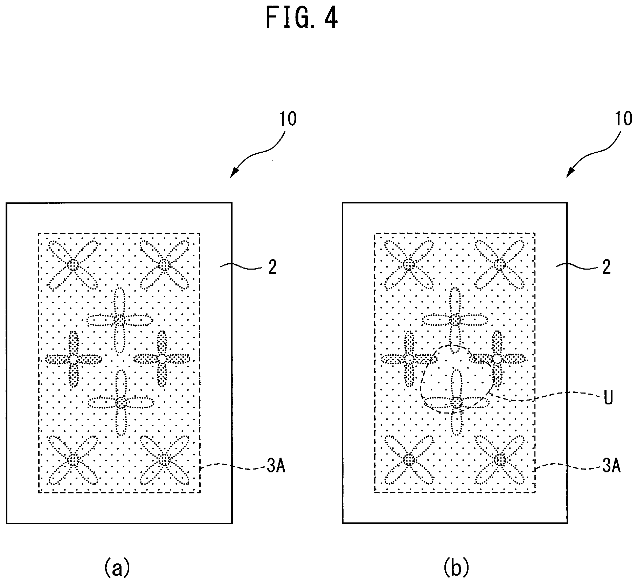

Fig. 4(a) is a plan view of an excreta treatment sheet for animals 10 according to another embodiment of the invention as viewed from the top sheet side, and Fig. 4(b) is a plan view schematically showing the state of the excreta treatment sheet for animals 10 of Fig. 4(a) after absorption of urine, as excreta.

The excreta treatment sheet for animals 10 of this different embodiment has the same construction as the excreta treatment sheet for animals 1 of the embodiment described above, except that as a colored sheet in the absorbent body 3A, this embodiment employs hydrophilic tissue paper that is colored with a single color shade overall and has colored regions with a flower-figure pattern (see Fig. 4(a)).

In the excreta treatment sheet for animals 10 of this different embodiment, when urine U that has been excreted from an animal is absorbed and held in the absorbent body 3A (especially the colored sheet and absorbent core), the color and outline of urine U that has been absorbed and held blends in with the color shade and flower pattern of the colored region of the colored sheet described above and is less conspicuous, as shown in Fig. 4(b), and therefore in addition to the concealing effect of the top sheet, urine U that has been absorbed and held in the absorbent body 3A is also less likely to be visible.

Incidentally, the sheet member composing the colored sheet is not particularly restricted, and any desired nonwoven fabric such as an air-through nonwoven fabric or spunbond nonwoven fabric may be used instead of the aforementioned tissue paper.

The absorbent core 32 in the excreta treatment sheet for animals 1 of the embodiment described above will now be explained in greater detail.

For this embodiment, the absorbent core 32 is composed of a first absorbent core 321 located on the top sheet side and the second absorbent core 322 located on the back sheet side, as explained above.

Moreover, as shown in Fig. 2, the first absorbent core 321 is composed of a particulate absorbent polymer 321a present in a planar dispersed state between the colored sheet 31 and the second absorbent core 322, and the second absorbent core 322 is composed of a mixture of a particulate absorbent polymer 322a and water-absorbent fibers 322b, disposed between the first absorbent core 321 and the core wrap sheet 33.

The absorbent polymer 321a composing the first absorbent core 321 and the absorbent polymer 322a composing the second absorbent core 322 may be the same absorbent polymer or different absorbent polymers. The absorbent polymer to be used for this embodiment is not particularly restricted so long as it can absorb and hold liquids, and any super-absorbent polymers may be employed, such as polyacrylic acid-based, starch-based or cellulosic types. The basis weight of the absorbent polymer is not particularly restricted, but from the viewpoint of liquid absorption properties, the basis weight of the absorbent polymer 321a composing the first absorbent core 321 is preferably in the range of 20 g/m² to 50 g/m², and the basis weight of the absorbent polymer 322a in the second absorbent core 322 is preferably in the range of 8 g/m ² to 30 g/m².

The water-absorbent fibers in the second absorbent core 322 are not particularly restricted so long as they can absorb and hold liquid, and for example, cellulosic water-absorbent fibers such as pulp (for example, fluff pulp) may be used.

The basis weight of the water-absorbent fibers in the first absorbent core is also not particularly restricted, but from the viewpoint of liquid absorption it is preferably in the range of 30 g/m² to 150 g/m².

Since the excreta treatment sheet for animals 1 of this embodiment has the absorbent core 32 of the absorbent body 3 composed of a first absorbent core 321 and a second absorbent core 322, excreta that has permeated the top sheet 2 pass between the absorbent polymer 321a of the first absorbent core 321 by affinity of the water-absorbent fibers 322b such as pulp in the second absorbent core 322, and are readily incorporated into the second absorbent core 322. Thus, since the excreta treatment sheet for animals 1 of this embodiment can rapidly absorb excreta such as urine into the absorbent body 3, the aforementioned concealing effect can be exhibited immediately after excretion by an animal, and a comfortable rearing environment can be provided for the owner of the animal. Moreover, since the excreta treatment sheet for animals 1 of this embodiment is less likely to have excreta such as urine residing between the top sheet 2 and the absorbent body 3, the legs and body hair of the animal using the excreta treatment sheet for animals 1 are less likely to become wetted.

Furthermore, if the absorbent core 32 in the absorbent body 3 is composed of the aforementioned first absorbent core 321 and second absorbent core 322, then even if excreta held between the water-absorbent fibers 322b in the second absorbent core 322 have seeped out by pressure from the legs of an animal such as a dog when the animal has stepped onto the excreta treatment sheet for animals 1 of this embodiment, the seeped out excreta can be absorbed by the absorbent polymer 322a in the second absorbent core 322 and the absorbent polymer 321a of the first absorbent core 321 located on the top sheet 2 side, such that excreta is less likely to return toward the first surface (the excreta supply surface) of the top sheet 2. This makes it is possible to prevent fouling of the legs of the animal, or generation of bad odor by excreta that have returned from the absorbent body 3.

In addition, in the excreta treatment sheet for animals 1 of this embodiment, the absorbent body 3 includes the absorbent polymer 321a of the first absorbent core 321 and the absorbent polymer 322a and water-absorbent fibers 322b in the second absorbent core 322, as absorbent materials, and therefore the water capacity of the absorbent body as a whole increases, and excreta that have been excreted from an animal can be absorbed and held reliably for prolonged periods.

Incidentally, for the embodiment described above, the absorbent core 32 in the absorbent body 3 is composed of the first absorbent core 321 and second absorbent core 322 as described above; however, according to the invention there is no limitation to such a structure, and the absorbent core may be composed of a single absorbent core as in the prior art, or it may be composed of three or more absorbent cores.

The absorbent material composing the absorbent core is also not limited to the aforementioned absorbent polymer or water-absorbent fibers, and any absorbent material conventionally used in the technical field may be employed.

For this embodiment, the core wrap sheet 33 is composed of similar tissue paper as the colored sheet 31 described above (for example, tissue paper with a basis weight of 13.0 g/m² to 13.5 g/m², formed using Northern bleached Kraft pulp as the main starting material), and in the absorbent body 3 it is disposed so as to cover the colored sheet 31 and absorbent core 32 from the back sheet side, preventing deformation of the absorbent core 32 or positional shifting of the colored sheet 31.

According to the invention, the core wrap sheet is not particularly restricted so long as it has liquid permeability and can prevent deformation of the absorbent core, and any desired nonwoven fabric such as an air-through nonwoven fabric or spunbond nonwoven fabric, for example, may be used instead of the aforementioned tissue paper.

The back sheet 4 to be used in the excreta treatment sheet for animals 1 of this embodiment will now be described in greater detail.

According to this embodiment, as shown in Fig. 2, the back sheet 4 is disposed facing the floor surface or ground on which the excreta treatment sheet for animals 1 is set in the thickness direction T of the excreta treatment sheet for animals 1, and it is made of a liquid-impermeable sheet member that functions to prevent leakage of excreta that have been discharged from an animal.

The liquid-impermeable sheet member composing the back sheet 4 is not particularly restricted, and for example, a non-air permeable resin film such as polyethylene or polypropylene, a laminate of a nonwoven fabric attached to a resin film, or a water-repellent or hydrophobic nonwoven fabric, may be suitably used.

The excreta treatment sheet for animals of the invention according to the embodiment described above can be produced in the following manner, for example.

### (1) Production of top sheet

Fibers (for example, PET (core)/PE (sheath) core-sheath composite fibers) containing a white pigment (for example, titanium oxide) in a prescribed content are supplied to a carding apparatus, and the core-sheath composite fibers are opened in the carding apparatus to form a web. The formed web is further transported downstream in the machine direction while being transported to an air-through system heating apparatus, for example, and the fibers in the web are tangled together in the heating apparatus to obtain a hydrophilic nonwoven fabric to serve as the top sheet.

When an uneven structure is to be formed on the first surface of the top sheet, for example, high-pressure air is uniformly blasted onto one surface (the first surface) of the hydrophilic nonwoven fabric to form the uneven structure across the entire surface of the first surface of the nonwoven fabric.

### (2) Production of absorbent body

An approximately flat second absorbent core is prepared containing the absorbent material, and a prescribed amount of absorbent polymer is evenly disposed on one surface of the second absorbent core to form a first absorbent core, to obtain an absorbent core comprising a laminate of the first absorbent core and the second absorbent core. After then attaching the colored sheet onto the top surface of the absorbent core, the colored sheet and absorbent core are covered by an optional core wrap sheet, to obtain an absorbent body.

### (3) Production of excreta treatment sheet for animals

A hot-melt adhesive, for example, may be used on one surface on the colored sheet side of the absorbent body to join the second surface of the top sheet, and then a polyethylene non-permeable sheet, for example, may be placed on the other surface on the second absorbent core side of the absorbent body, as a back sheet, and the absorbent body inserted between the top sheet and the back sheet while joining the perimeters of the top sheet and the back sheet with a hot-melt adhesive, to obtain an excreta treatment sheet for animals of the invention, such as the embodiment described above.

The excreta treatment sheet for animals of the invention is not restricted to the different embodiments described above or the examples described below, and it may incorporate appropriate combinations and modifications in a range that is not outside of the object and gist of the invention. For example, according to another embodiment of the invention, the excreta treatment sheet for animals may comprise an interlayer sheet having diffusibility for liquids in the lengthwise direction L and/or the widthwise direction W, either between the top sheet and the absorbent body or between the absorbent body and the back sheet, or both. If the excreta treatment sheet for animals comprises such an interlayer sheet, excreta can be absorbed into the absorbent body while diffusing in the lengthwise direction L and/or the widthwise direction W by the interlayer sheet, thereby allowing excellent absorption efficiency and high absorption performance, including resistance to rewetting, to be exhibited in addition to the aforementioned excellent masking performance.

Incidentally, the ordinal terms "first" and "second" as used throughout the present description serve merely to distinguish between the numbered embodiments and are not used to mean any relative ordering, precedence or importance.

### Examples

The invention will now be explained in greater detail using examples and comparative examples, with the understanding that the invention is not limited only to these examples.

### Example 1

PET (core)/PE (sheath) core-sheath composite fibers (size: 2.2 dtex, fiber length: 51 mm) containing 2.40 mass% of titanium oxide as a white pigment in the core, were supplied to a carding apparatus, and the core-sheath composite fibers were opened in the carding apparatus to form a web. The formed web was further transported downstream in the machine direction while being transported to an air-through system heating apparatus, and the fibers in the web were tangled together in the heating apparatus to obtain a hydrophilic nonwoven fabric comprising a top sheet according to Example 1. The top sheet of Example 1 obtained in this manner had a basis weight of 22 g/m² and a thickness of 0.99 mm.

### Example 2

A top sheet for Example 2 was fabricated in the same manner as Example 1, except that the titanium oxide content in the core of the core-sheath composite fibers was 2.43 mass%, and the basis weight and thickness of the top sheet were 25 g/m² and 1.30 mm, respectively.

### Example 3

A top sheet for Example 3 was fabricated in the same manner as Example 1, except that basis weight and thickness of the top sheet were 20 g/m ² and 0.77 mm, respectively.

### Comparative Example 1

A top sheet for Comparative Example 1 was fabricated in the same manner as Example 1, except that the titanium oxide content in the core of the core-sheath composite fibers was 2.43 mass%, the basis weight and thickness of the top sheet were 25 g/m ² and 1.30 mm, respectively, and a ridge-furrow structure was formed on the first surface of the top sheet, composed of a plurality of ridges extending in one direction and furrows formed between the adjacent ridges.

### Comparative Example 2

A top sheet for Comparative Example 2 was fabricated in the same manner as Example 1, except that the titanium oxide content in the core of the core-sheath composite fibers was 0.45 mass%, and the basis weight and thickness of the top sheet were 25 g/m ² and 1.29 mm, respectively.

### Comparative Example 3

A top sheet for Comparative Example 3 was fabricated in the same manner as Example 1, except that the titanium oxide content in the core of the core-sheath composite fibers was 0.04 mass%, and the basis weight and thickness of the top sheet were 17 g/m² and 0.18 mm, respectively.

### Comparative Example 4

A top sheet for Comparative Example 4 was fabricated in the same manner as Example 1, except that the titanium oxide content in the fibers composing the top sheet was ≤0.04 mass%, and the basis weight and thickness of the top sheet were 8 g/m ² and 0.13 mm, respectively.

### Comparative Example 5

A top sheet for Comparative Example 5 was fabricated in the same manner as Example 1, except that the titanium oxide content in the fibers composing the top sheet was ≤0.04 mass%, and the basis weight and thickness of the top sheet were 17 g/m² and 0.65 mm, respectively.

Each of the top sheets of Examples 1 to 3 and Comparative Examples 1 to 5, obtained as described above, were measured for mean absorbance, mean flock value and flock ratio on the first surface, by the following measuring methods. In addition, the masking properties of each of the top sheets of Examples 1 to 3 and Comparative Examples 1 to 5 were evaluated by the visibility evaluation methods described below. The measurement results and evaluation results are shown in Table 1.

### [Measuring methods for mean absorbance, mean flock value and flock ratio]

(1) A 30 cm × 20 cm test piece is obtained from the top sheet to be measured.
(2) Using a Formation Tester (Model No. FMT-MIII by Nomura Shoji Co., Ltd.), irradiation of light is carried out with a tungsten current at a constant direct current (6V, 30W), from the lower side of the test piece set on a diffuser panel.
(3) A transmission image taken in a 25 cm × 18 cm range with a CCD camera is resolved to 320 × 230 (= 73,600) pixels, the intensity of light received by each pixel is measured and the mean absorbance, the flock value in the front-back direction (first direction) and the flock value in the left-right direction (second direction) are each calculated. The other measuring conditions are 10 bits for the measured value of each pixel, 1 average moving pixel, and 1 flock treatment.
(4) Based on the obtained first direction flock value and second direction flock value, the sum of both values is divided by 2 to calculate the average flock value, and of the first direction flock value and the second direction flock value, the smaller flock value is divided by the larger flock value to calculate the flock ratio.

### [Visibility evaluation method]

(1) The top sheet to be evaluated is used to cover an optional absorbent body including blue-colored tissue paper, to fabricate an evaluation sample.
(2) A prescribed amount of yellow-colored water as artificial urine is dropped onto the top sheet of the evaluation sample.
(3) The aspect of visibility of the artificial urine by visual inspection from the top sheet side after absorption of the artificial urine in the absorbent body is assessed on the scale shown below.
(4) Steps (1) to (3) are carried out 7 times, and the average is calculated as the assessment value.

### (Assessment scale)

4 points: Almost no visibility of artificial urine
3 points: Low visibility of artificial urine
2 points: Visibility of artificial urine
1 point: High visibility of artificial urine
0 points: Clear visibility of artificial urine

### [Table 1]

**Table 1**

| | | Example 1 | Example 2 | Example 3 | Comp. Example 1 | Comp. Example 2 | Comp. Example 3 | Comp. Example 4 | Comp. Example 5 |
|---|---|---|---|---|---|---|---|---|---|
| Titanium oxide content (mass%) | | 2.40 | 2.43 | 2.40 | 2.43 | 0.45 | 0.04 | ≤0.04 | ≤0.04 |
| Basis weight (g/m²) | | 22 | 25 | 20 | 25 | 25 | 17 | 8 | 17 |
| Thickness (mm) | | 0.99 | 1.30 | 0.77 | 1.30 | 1.29 | 0.18 | 0.13 | 0.65 |
| First surface measurement results | Mean absorbance | 879 | 1073 | 807 | 1040 | 508 | 528 | 294 | 290 |
| | First direction flock value | 3.88 | 3.58 | 4.01 | 3.16 | 3.29 | 2.98 | 3 | 2.66 |
| | Second direction flock value | 5.06 | 5.24 | 5.47 | 6.14 | 4.25 | 3.43 | 2.83 | 3.26 |
| | Mean flock value | 4.47 | 4.41 | 4.74 | 4.65 | 3.77 | 3.19 | 2.92 | 2.96 |
| | Flock ratio | 0.77 | 0.68 | 0.73 | 0.51 | 0.77 | 0.88 | 1.06 | 0.82 |
| | Transmission image | | | | | | | | |
| Visibility evaluation results | 1 | 4 | 4 | 3 | 2 | 2 | 1 | 0 | 1 |
| | 2 | 3 | 4 | 2 | 3 | 2 | 1 | 0 | 0 |
| | 3 | 3 | 3 | 3 | 2 | 2 | 2 | 1 | 1 |
| | 4 | 3 | 3 | 3 | 2 | 1 | 1 | 1 | 2 |
| | 5 | 4 | 4 | 2 | 3 | 2 | 1 | 2 | 1 |
| | 6 | 4 | 3 | 3 | 2 | 2 | 1 | 0 | 1 |
| | 7 | 4 | 3 | 3 | 2 | 2 | 1 | 1 | 2 |
| | Assessment value (Average value) | 4 | 3 | 3 | 2 | 2 | 1 | 1 | 1 |

As shown in Table 1, the top sheets of Examples 1 to 3 satisfying the aforementioned specific requirements in regard to mean absorbance, mean flock value and flock ratio had more excellent masking properties after absorption of artificial urine, compared to the top sheets of Comparative Examples 1 to 5 that did not satisfy the specific requirements.

### Reference Sign List

1 Excreta treatment sheet for animals
2 Top sheet
3 Absorbent body
31 Colored sheet
32 Absorbent core
321 First absorbent core
322 Second absorbent core
33 Core wrap sheet
4 Back sheet

## Claims

1. An excreta treatment sheet for animals comprising a liquid-permeable top sheet composed of white fibers, a liquid-impermeable back sheet and an absorbent body disposed between the sheets, wherein
the top sheet has a first surface serving as an excreta supply surface and a second surface that is the surface on the side opposite the first surface, and
the top sheet has a mean absorbance of 700 or greater on the first surface, a mean flock value of no greater than 6.0 on the first surface, and a flock ratio of 0.6 to 1.7 on the first surface.

2. The excreta treatment sheet for animals according to claim 1, wherein the top sheet has a mean absorbance of no greater than 2000 on the first surface.

3. The excreta treatment sheet for animals according to claim 1 or 2, wherein the top sheet has an uneven structure on the first surface and a flat structure on the second surface.

4. The excreta treatment sheet for animals according to any one of claims 1 to 3, wherein the white fibers include titanium oxide, and content of the titanium oxide in the fibers is 1 to 3 mass%.

5. The excreta treatment sheet for animals according to claim 4, wherein the white fibers are composed of core-sheath thermoplastic resin fibers, and the titanium oxide is present only in cores of the core-sheath thermoplastic resin fibers.

6. The excreta treatment sheet for animals according to any one of claims 1 to 5, wherein thickness of the top sheet is 0.5 mm or greater.

7. The excreta treatment sheet for animals according to any one of claims 1 to 6,
wherein basis weight of the top sheet is 20 g/m ² or greater.

8. The excreta treatment sheet for animals according to any one of claims 1 to 7, wherein
the absorbent body has an absorbent core including an absorbent material, and
a colored sheet at least partially having colored regions is disposed between the top sheet and the absorbent core.
